# DEMANDE DE BREVET EUROPEEN

(11) **EP 1 961 455 A1**
(43) Date de publication de la demande: **27.08.2008**
(21) Numéro de dépôt: 08151252.7
(22) Date de dépôt: 11.02.2008
(51) Int. Cl.: A61Q 19/10, A61K 8/894

(54) **Emulsion e/h pour le soin de la peau**

(30) Priorité: 21.02.2007 FR 0753392
(71) Demandeur: L'Oréal, 75008 Paris (FR)
(72) Inventeur: Lorant, Raluca, 94320, THIAIS (FR)
(74) Mandataire: Duvert, Sandra

(57) **Abrégé**

L'invention se rapporte à une composition pour application topique sous forme d'émulsion eau-dans-huile contenant au moins un élastomère de silicone émulsionnant et au moins un polymère semi-cristallin.

L'invention se rapporte aussi à l'utilisation de la composition selon l'invention pour le soin de la peau, et notamment pour nourrir la peau, prévenir la perte d'eau transépidermique et protéger la peau.

## Description

L'invention se rapporte à une composition cosmétique se présentant sous forme d'une émulsion eau-dans-huile (E/H) comportant une quantité importante de phase aqueuse, et contenant un organopolysiloxane élastomère oxyalkyléné ou glycérolé et un polymère semi-cristallin. L'invention se rapporte aussi aux utilisations de cette composition, en particulier comme produit de soin de la peau.

Dans le domaine cosmétique, les émulsions de type eau-dans-huile (E/H) sont reconnues comme étant efficaces pour le soin de la peau du fait qu'elles comportent une phase continue huileuse et qu'elles permettent donc de former à la surface de la peau, un film lipidique qui prévient la perte d'eau transépidermique et protège la peau des agressions extérieures. Ces émulsions sont particulièrement appropriées pour protéger et nourrir la peau, et en particulier pour traiter les peaux sèches. Toutefois, il est souvent reproché aux émulsions E/H d'être trop grasses et collantes et de présenter une stabilité fragile. Généralement, elles n'apportent pas de fraîcheur et elles sont souvent trop riches en huiles pour être utilisées pendant l'été ou dans les pays chauds.

Pour surmonter ces inconvénients, il a été proposé de préparer des émulsions E/H à forte teneur en phase aqueuse et en eau. Ainsi, le document EP-A-1,068,851 décrit l'utilisation d'un organopolysiloxane élastomère oxyalkyléné comme émulsionnant pour stabiliser des émulsions E/H contenant au moins 70% de phase aqueuse. Toutefois, les compositions obtenues selon ce document, bien qu'apportant effectivement une certaine fraîcheur à l'application grâce au taux de phase aqueuse très important, n'apportent pas les bénéfices connues des émulsions E/H, telle que la nutrition des peaux sèches, du fait de la nature volatile des huiles qu'elles contiennent. En effet, les huiles volatiles, par définition, s'évaporent et, si elles apportent une impression agréable de fraîcheur à l'étalement, elles n'apportent pas d'effet traitant puisqu'elles ne restent pas sur la peau. De plus, la nature chimique de l'émulsionnant siliconé impose des limites dans la nature des huiles composant la phase huileuse : pour des raisons de faisabilité et stabilité, elle doit contenir majoritairement des huiles siliconées et/ou des huiles volatiles qui doivent représenter au moins 15% en poids du poids total de la composition. En outre, les émulsions décrites dans ce document nécessitent la présence d'un électrolyte dans la phase aqueuse pour assurer un niveau de stabilité acceptable. Or certaines peaux particulièrement réactives peuvent mal tolérer des compositions contenant une quantité d'électrolytes telle que celles décrites dans les exemples de ce document (2,5 %).

Par ailleurs, le document EP-A-1,136,058 propose également des émulsions E/H apportant de la fraîcheur à l'application sur la peau, grâce à la présence d'un taux élevé de phase aqueuse. Toutefois, comme dans le document précédemment cité, ces compositions stabilisées par un organopolysiloxane élastomère oxyalkyléné posent les mêmes problèmes, à savoir que la présence d'huiles volatiles notamment siliconées est en proportion prédominante.

Il subsiste donc le besoin d'une composition sous forme d'émulsion E/H qui ne présente pas les inconvénients de l'art antérieur et notamment qui procure :
- un effet de fraîcheur à l'application, notamment grâce à la présence d'un taux élevé de phase aqueuse,
- un effet de nutrition, qui est le bénéfice premier des émulsions à phase continue huileuse,
tout en montrant une bonne stabilité dans le temps.

La demanderesse a maintenant trouvé de manière surprenante que l'on pouvait obtenir une composition ayant les avantages recherchés et pouvant contenir des huiles non volatiles, en utilisant une émulsion eau dans huile contenant un élastomère de silicone émulsionnant et un polymère lipophile particulier. Par « élastomère de silicone émulsionnant », on entend un élastomère de silicone comprenant au moins une chaîne hydrophile, ce qui lui confère des propriétés émulsionnantes.

L'invention a pour objet une composition pour application topique sous forme d'émulsion eau-dans-huile comprenant une phase aqueuse dispersée dans une phase huileuse, et contenant au moins un élastomère de silicone émulsionnant et au moins un polymère semi-cristallin.

La composition étant pour une application topique, elle contient un milieu physiologiquement acceptable. On entend par « milieu physiologiquement acceptable », un milieu non toxique, compatible avec la peau (y compris l'intérieur des paupières), les muqueuses, les cheveux ou les lèvres d'êtres humains. La composition constituant de préférence une composition cosmétique, elle a un aspect, une odeur et un toucher agréables.

La composition de l'invention présente l'avantage de donner un effet de fraîcheur lors de l'application sur la peau, tout en ayant une bonne efficacité de nutrition de la peau et tout en montrant une bonne stabilité dans le temps. En outre, elle peut contenir une quantité importante d'huiles non volatiles sans être instable.

### Elastomère de silicone émulsionnant

Par "élastomère de silicone", on entend un organopolysiloxane partiellement ou totalement réticulé, matériau souple et déformable ayant des propriétés viscoélastiques. Son module d'élasticité est tel que ce matériau résiste à la déformation et possède une capacité limitée à l'extension et à la contraction. Ce matériau est capable de retrouver sa forme originelle suite à un étirement.

L'élastomère de silicone émulsionnant utilisé selon l'invention est un organopolysiloxane élastomère réticulé comprenant au moins une chaîne hydrophile, cette chaîne pouvant être notamment oxyalkylénée ou glycérolée. L'élastomère de silicone émulsionnant peut donc être choisi parmi les élastomères de silicone comportant au moins une chaîne oxyalkylénée et/ou une chaîne glycérolée.

L'élastomère de silicone comportant au moins une chaîne oxyalkylénée peut être obtenu notamment par réaction d'addition et réticulation d'un diorganopolysiloxane contenant au moins deux hydrogènes liés chacun à un silicium (A1), et d'un polyoxyalkylène ayant au moins deux groupements à insaturation éthylénique (B1), notamment en présence d'un catalyseur (C1), notamment de catalyseur platine, comme par exemple décrit dans les documents US-A-5,236,986 et US-A-5,412,004.

Le composé (A1) est le composé de base pour la formation d'organopolysiloxane élastomère, et la réticulation s'effectue par réaction d'addition du composé (A1) avec le composé (B1) en présence du catalyseur (C1).

Le composé (B1) est avantageusement un composé oxyéthyléné et/ou oxypropyléné comportant au moins deux groupes vinyliques en position α-ω de la chaîne siliconée, qui vont réagir avec des liaisons Si-H du composé (A1). Le composé (B1) peut être notamment un polyoxyalkylène (notamment polyoxyéthyléne et/ou polyoxypropylène) à terminaisons diméthylvinylsiloxy.

Les groupes organiques liés aux atomes de silicium du composé (A1) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto.

Le composé (A1) peut ainsi être choisi parmi les méthylhydrogénopolysiloxanes à terminaisons triméthylsiloxy, les copolymères diméthylsiloxaneméthyl-hydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (C1) est le catalyseur de la réaction de réticulation, et est notamment choisi parmi l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C1) est de préférence ajouté en une quantité de 0,1 à 1000 parts en poids, mieux de 1 à 100 parts en poids, en tant que métal platine propre pour 1000 parts en poids de la quantité totale des composés (A1) et (B1).

En particulier, l'élastomère de silicone comportant au moins une chaîne oxyalkylénée peut être obtenu par réaction de polyoxyalkylène (notamment polyoxyéthyléne et/ou polyoxypropylène) à terminaisons diméthylvinylsiloxy et de méthylhydrogénopolysiloxane à terminaisons triméthylsiloxy, en présence de catalyseur platine.

L'élastomère de silicone comportant au moins une chaîne oxyalkylénée, utilisé selon l'invention, est de préférence un élastomère de silicone comportant au moins une chaîne oxyéthylénée.

En outre, l'élastomère de silicone comportant au moins une chaîne oxyalkylénée est de préférence véhiculé sous forme de gel dans au moins une huile hydrocarbonée et/ou une huile siliconée. Dans ces gels, l'élastomère comportant au moins une chaîne oxyalkylénée est souvent sous forme de particules non-sphériques.

Des élastomères de silicone polyoxyalkylénés sont notamment décrits dans les documents US-A-5,236,986, US-A-5,412,004, US-A-5,837,793, US-A-5,811,487 dont le contenu est incorporé par référence.

Comme élastomères de silicone comportant au moins une chaîne oxyéthylénée, on peut utiliser notamment ceux commercialisés par la société Shin Etsu sous les dénominations :
- KSG-21 (à 27 % en matière active. Nom INCI : Dimethicone /PEG-1 0 Dimethicone vinyl dimethicone crosspolymer),
- KSG-20 (à 100 % en matière active. Nom INCl : PEG-10 Dimethicone Crosspolymer),
- KSG-210 (à 25 % en matière active. Nom INCl: Dimethicone /PEG-10/15 crosspolymer),
- X-22-6146 (à 32 % en matière active. Nom INCl : Dimethicone /PEG-10 Dimethicone vinyl dimethicone crosspolymer),
ou ceux commercialisés par la société Dow Corning sous les dénominations:
- DC9010 (à 11 % en matière active. Nom INCI : PEG-12 dimethicone crosspolymer)
- DC9011 (à 9 1% en matière active).

Ces produits se présentent généralement sous forme de gels huileux contenant les particules d'élastomère de silicone.

On utilise de préférence le KSG-210 (Nom INCl : Dimethicone /PEG-10/15 crosspolymer) qui est à 25% en matière active d'élastomère de silicone dans de l'huile de silicone.

L'élastomère de silicone émulsionnant peut être également choisi parmi les élastomères de silicone comportant au moins une chaîne glycérolée.

L'élastomère de silicone comportant au moins une chaîne glycérolée peut être obtenu notamment par réaction d'addition et réticulation d'un diorganopolysiloxane contenant au moins un hydrogène lié au silicium (A2) et d'un composé polyglycérolé ayant des groupements à insaturation éthylénique (B2), notamment en présence de catalyseur (C2), notamment de catalyseur platine.

En particulier, l'organopolysiloxane peut être obtenu par réaction de composé polyglycérolé à terminaisons diméthylvinylsiloxy et de méthylhydrogéno-polysiloxane à terminaisons triméthylsiloxy, en présence de catalyseur platine.

Le composé (A2) est le composé de base pour la formation d'organopolysiloxane élastomère, et la réticulation s'effectue par réaction d'addition du composé (A) avec le composé (B2) en présence du catalyseur (C2).

Le composé (A2) est en particulier un organopolysiloxane ayant au moins 2 atomes d'hydrogène liés à des atomes de silicium distincts dans chaque molécule. Le composé (A2) peut avoir une viscosité à 25 °C allant de 1 à 50 000 centistokes, notamment pour être bien miscible avec le composé (B2).

Les groupes organiques liés aux atomes de silicium du composé (A2) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl, ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto. De préférence, ledit groupe organique est choisi parmi les groupes méthyl, phényl, lauryl.

Le composé (A2) peut ainsi être choisi parmi les méthylhydrogénopolysiloxanes à terminaisons triméthylsiloxy, les copolymères diméthylsiloxaneméthyl-hydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (B2) peut être un composé polyglycérolé répondant à la formule (B'2) suivante :

CₘH₂ₘ₋₁-O-[Gly ]ₙ- CₘH₂ₘ₋₁ (B'2)

dans laquelle m est un entier allant de 2 à 6, n est un entier allant de 2 à 200, de préférence de 2 à 100, préférentiellement de 2 à 50, mieux de 2 à 20, encore mieux de 2 à 10, et encore mieux de 2 à 5, et en particulier n est égal à 3 ; Gly désigne :

-CH₂-CH(OH)-CH₂-O- ou -CH₂-CH(CH₂OH)-O-

Avantageusement, la somme du nombre de groupements éthyléniques par molécule du composé (B2) et du nombre d'atomes d'hydrogène liés à des atomes de silicium par molécule du composé (A2) est d'au moins 4.

Il est avantageux que le composé (A2) soit ajouté en une quantité telle que le rapport moléculaire entre la quantité totale d'atomes d'hydrogène liés à des atomes de silicium dans le composé (A2) et la quantité totale de tous les groupements à insaturation éthylénique dans le composé (B2) soit compris dans la gamme de 1 /1 à 20/1.

Le composé (C2) est le catalyseur de la réaction de réticulation, et est notamment choisi parmi l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C2) est de préférence ajouté en une quantité de 0,1 à 1000 parts en poids, mieux de 1 à 100 parts en poids, en tant que métal platine propre pour 1000 parts en poids de la quantité totale des composés (A2) et (B2).

L'élastomère de silicone comportant au moins une chaîne glycérolée, utilisé selon l'invention se présente généralement sous forme d'un gel en mélange avec au moins une huile hydrocarbonée et/ou une huile siliconée. Dans ces gels, l'élastomère comportant au moins une chaîne glycérolée est souvent sous forme de particules non-sphériques.

De tels élastomères sont notamment décrits dans le document WO-A-2004/024798.

Comme élastomère de silicone comportant au moins une chaîne glycérolée, on peut utiliser celui vendu par la société Shin Etsu sous la dénomination, KSG-710 (à 25% en matière active. Nom INCl : Dimethicone / Polyglycerin-3 Crosspolymer).

De façon préférentielle, la quantité (en matière active) d'élastomère(s) de silicone émulsionnant(s) dans la composition de l'invention va de 0,1 à 20 % en poids, mieux de 0,5 à 16 % en poids, encore mieux de 0,5 à 10 % en poids et encore mieux de 0,5 à 5 % en poids par rapport au poids total de la composition.

L'élastomère de silicone émulsionnant est généralement introduit dans la phase huileuse. et fait partie de cette phase huileuse.

### Polymères semi-cristallins

La composition selon l'invention contient au moins un polymère semi-cristallin, de préférence dérivé d'acide acrylique ou méthacrylique. Par "polymère semi-cristallin", on entend au sens de l'invention, des polymères comportant une partie cristallisable, chaîne pendante ou séquence dans le squelette, et une partie amorphe dans le squelette, et présentant une température de changement de phase réversible du premier ordre, en particulier de fusion (transition solide-liquide). Lorsque la partie cristallisable est une séquence du squelette polymérique, cette séquence cristallisable est de nature chimique différente de celle des séquences amorphes ; le polymère semi-cristallin est dans ce cas un polymère séquencé par exemple du type dibloc, tribloc ou multibloc.

De façon avantageuse, le ou les polymères semi-cristallins de la composition de l'invention ont une masse moléculaire moyenne en nombre Mn supérieure ou égale à 2000, allant par exemple de 2 000 à 800 000, de préférence de 3 000 à 500 000, par exemple de 4 000 à 150 000, et mieux de 4 000 à 99 000.

Dans la composition selon l'invention, les polymères semi-cristallins sont avantageusement solubles dans la phase huileuse à au moins 1 % en poids, à une température supérieure à leur température de fusion. En dehors des chaînes ou séquences cristallisables, les séquences des polymères sont amorphes. Par "chaîne ou séquence cristallisable", on entend au sens de l'invention une chaîne ou séquence qui, si elle était seule, passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitifs du polymère.

De préférence, le squelette polymérique des polymères semi-cristallins est soluble dans la phase huileuse.

De façon préférée, les polymères semi-cristallins utilisés dans la composition de l'invention présentent une température de fusion (ou point de fusion), pF, inférieure à 70°C (25°C ≤pF< 70°C), cette température étant au moins égale à la température de la matière kératinique devant recevoir la composition selon l'invention, notamment la peau. La température de fusion peut être mesurée notamment par toute méthode connue et en particulier avec un calorimètre à balayage différentiel (D.S.C).

De préférence, les séquences ou chaînes cristallisables des polymères semi-cristallins représentent au moins 30 % du poids total de chaque polymère et mieux au moins 40 %. Les polymères semi-cristallins à séquences cristallisables utilisés selon l'invention sont des polymères séquencés ou multiséquencés. Ils peuvent être obtenus par polymérisation de monomères à doubles liaisons réactives (ou éthyléniques) ou par polycondensation. Lorsque les polymères de l'invention sont des polymères à chaînes latérales cristallisables, ils sont avantageusement sous forme aléatoire ou statistique.

Les polymères semi-cristallins de l'invention sont d'origine synthétique. En outre, ils ne comportent pas de squelette polysaccharidique.

Les polymères semi-cristallins utilisables dans l'invention sont de préférence choisis parmi les polymères (homopolymères ou copolymères) portant au moins une chaîne latérale cristallisable, et les polymères (homopolymères ou copolymères) portant dans le squelette au moins une séquence cristallisable, comme ceux décrits dans le document US-A-5,156,911. La ou les chaînes latérales ou séquences cristallisables sont hydrophobes.

Selon un mode préféré de réalisation de l'invention, les polymères semi-cristallins sont choisis notamment parmi les homopolymères et copolymères résultant de la polymérisation d'au moins un monomère à chaîne(s) cristallisable(s), celle-ci étant choisie parmi les chaînes alkyles comportant au moins 11 atomes de carbone et au plus 40 atomes de carbone et mieux au plus 24 atomes de carbone. Il s'agit notamment de chaînes alkyle comportant au moins 12 atomes de carbone, et de préférence, il s'agit de chaînes alkyle comportant de 14 à 24 atomes de carbone (C₁₄-C₂₄). Il peut s'agir de chaînes alkyle hydrocarbonées (atomes de carbone et d'hydrogène) ou chaînes alkyle fluorées ou perfluorées (atomes de carbone, atomes de fluor et éventuellement atomes d'hydrogène). Lorsqu'il s'agit de chaînes alkyle fluorées ou perfluorées, elles comportent au moins 11 atomes de carbone dont au moins 6 atomes de carbone sont fluorés.

Par "alkyle", on entend au sens de l'invention un groupement saturé (ne comportant pas d'insaturation).

Selon un mode particulier de réalisation de l'invention, le polymère semi-cristallin est choisi parmi les homopolymères obtenus par polymérisation d'au moins un monomère à chaîne cristallisable, choisi parmi les (méth)acrylates d'alkyle en C₁₄-C₂₄, les (méth)acrylates de perfluoroalkyle en C₁₁-C₁₅, les N alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alpha-oléfines en C₁₄ à C₂₄, les para-alkyl styrènes avec un groupe alkyle en C₁₄ à C₂₄, et parmi les copolymères de ces monomères, obtenus par copolymérisation de ces monomères avec un monomère hydrophile, de préférence différent de l'acide méthacrylique, comme par exemple la N-vinylpyrrolidone, l'hydroxyéthylacrylate, l'hydroxyéthylméthacrylate, l'acide acrylique. De tels copolymères peuvent être par exemple les copolymères d'alkyl-C₁₄-C₂₄-acrylate, d'alkyl-C₁₄-C₂₄-méthacrylate, d'alkyl-C₁₄-C₂₄-acrylamide, d'alkyl-C₁₄-C₂₄-méthacrylamide, avec la N-vinylpyrrolidone, l'hydroxyéthylacrylate, l'hydroxyéthyl-méthacrylate, l'acide acrylique, ou leurs mélanges.

De préférence , le polymère semi-cristallin est choisi parmi les homopolymère obtenus par polymérisation d'un monomère choisi parmi les acrylates d'alkyle en C₁₄-C₂₄ et les méthacrylates d'alkyle en C₁₄-C₂₄ et parmi les copolymères obtenus par copolymérisation d'un monomère choisi parmi les acrylates d'alkyle en C₁₄-C₂₄ et les méthacrylates d'alkyle en C₁₄-C₂₄, avec un monomère hydrophile tel que l'acide acrylique.

Les polymères semi-cristallins de la composition de l'invention peuvent être non réticulés ou réticulés en partie, du moment que le taux de réticulation ne gène pas leur dissolution ou dispersion dans la phase huileuse par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère, comme par exemple les interactions dipolaires entre ionomères carboxylates, ces interactions étant en faible quantité et portées par le squelette du polymère ; soit à une séparation de phase entre les séquences cristallisables et les séquences amorphes portées par le polymère.

De préférence, les polymères semi-cristallins de la composition selon l'invention sont non réticulés.

Selon un mode particulier de réalisation de l'invention, le polymère semi-cristallin est un homopolymère résultant de la polymérisation d'un monomère à chaîne cristallisable choisi parmi les acrylates d'alkyle en C₁₄-C₂₄ et les méthacrylates d'alkyle en C₁₄-C₂₄. On peut citer notamment ceux commercialisés sous les dénominations Intelimer® par la société Landec, décrits dans la brochure "Intelimer® polymers", Landec IP22. Ces polymères sont sous forme solide à température ambiante. Ils portent des chaînes latérales cristallisables et correspondent à des homopolymères d'acrylates ou méthacrylates d'alkyle en C₁₄-C₂₄ saturé. On peut citer plus particulièrement l'homopolymère d'acrylate de stéaryle (Intelimer IPA-13.1) (nom INCl : Poly C10-30 alkyl acrylate), l'homopolymère d'acrylate de béhényle (Intelimer IPA-13.6) (nom INCl : Poly C10-30 alkyl acrylate).

Selon un autre mode particulier de réalisation de l'invention, le polymère semi-cristallin est un copolymère d'acrylates d'alkyle en C₁₄-C₂₄ ou de méthacrylates d'alkyle en C₁₄-C₂₄ avec l'acide acrylique Comme copolymères de ce type, on peut citer les copolymères obtenus par la copolymérisation de l'acrylate de béhényle et de l'acide acrylique, et les copolymères obtenus par la copolymérisation de l'acrylate de stéaryle et de l'acide acrylique.

Selon un mode préféré de réalisation de l'invention, le polymère semi-cristallin est un homopolymère, et il est choisi parmi l'homopolymère d'acrylate de stéaryle (Intelimer IPA-13.1) (nom INCI : Poly C10-30 alkyl acrylate), l'homopolymère d'acrylate de béhényle (Intelimer IPA-13.6) (nom INCI : Poly C10-30 alkyl acrylate), et leurs mélanges.

La quantité de polymère(s) semi-cristallin(s) dans la composition de l'invention peut aller par exemple de 0,1 à 5 % en poids, de préférence de 0,5 à 5 % en poids et mieux de 1 à 4 % en poids par rapport au poids total de la composition.

### Polymères d'acide 2-acrylamido 2-méthylpropane sulfonique

Selon un mode particulier de réalisation de l'invention, la composition peut contenir en outre un ou plusieurs polymères d'acide 2-acrylamido 2-méthylpropane sulfonique. L'addition d'un tel polymère présente l'avantage de conforter la stabilité des émulsions même en absence d'électrolyte.

On entend dans la présente demande « polymère comportant des motifs acide 2-acrylamido 2-méthylpropane sulfonique » (AMPS), aussi bien des homopolymères que des copolymères, et aussi bien des polymères réticulés que des polymères non réticulés.

Ce sont des polymères hydrosolubles ou hydrodispersibles ou gonflables dans l'eau.

De façon préférentielle, les polymères d'AMPS utilisés conformément à l'invention peuvent être neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés. Ils sont généralement neutralisés. On entend dans la présente invention par « neutralisés », des polymères totalement ou pratiquement totalement neutralisés, c'est-à-dire neutralisés à au moins 90 %.

Les polymères d'AMPS utilisés dans la composition de l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Lorsque les polymères sont réticulés, les agents de réticulation peuvent être choisis parmi les composés à polyinsaturation oléfinique couramment utilisés pour la réticulation des polymères obtenus par polymérisation radicalaire. On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés. Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10 % en mole et plus particulièrement de 0,2 à 2 % en mole par rapport au polymère.

Les homopolymères d'AMPS préférés pour être utilisés dans la composition de l'invention sont réticulés et neutralisés, et ils peuvent être obtenus selon le procédé de préparation comprenant les étapes suivantes :
(a) on disperse ou on dissout l'acide 2-acrylamido 2-méthylpropane sulfonique sous forme libre dans une solution de tertio-butanol ou d'eau et de tertio-butanol ;
(b) on neutralise la solution ou la dispersion de monomère obtenue en (a) par une ou plusieurs bases minérales ou organiques, de préférence l'ammoniaque NH₃, dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants ;
(d) on effectue une polymérisation radicalaire classique en présence d'amorceurs de radicaux libres à une température allant de 10 à 150°C ; le polymère précipitant dans la solution ou la dispersion à base de tertio-butanol.

Les homopolymères d'AMPS plus particulièrement préférés comprennent, distribués de façon aléatoire, des motifs de formule générale (I) suivante : 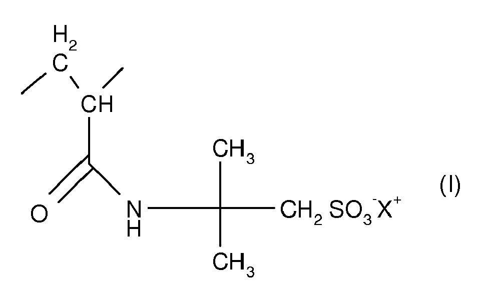 dans laquelle X⁺ désigne un proton, un cation de métal alcalin, un cation alcalino-terreux ou l'ion ammonium, au plus 10% mol des cations X⁺ pouvant être des protons H⁺ ;
et des motifs réticulants provenant d'au moins un monomère ayant au moins deux double-liaison oléfiniques.

Les homopolymères utilisés selon l'invention et plus particulièrement préférés comprennent de 90 à 99,9% en poids et de préférence de 98 à 99,5 % en poids de motifs de formule (I), et de 0,01 à 10% en poids, de préférence de 0,2 à 2 % en poids de motifs réticulants, les proportions en poids étant définis par rapport au poids total du polymère.

Comme homopolymère de ce type, on peut citer notamment l'homopolymère réticulé et neutralisé d'acide 2-acrylamido 2-méthylpropane sulfonique, commercialisé par la société Clariant sous la dénomination commerciale « Hostacerin AMPS » (nom INCI : Ammonium Polyacryldimethyltauramide).

Les copolymères d'acide 2-acrylamido-2-méthylpropane sulfonique (AMPS) utilisables dans la composition de l'invention peuvent être choisis notamment parmi :
1) les copolymères anioniques réticulés d'acrylamide ou méthylacrylamide et d'acide 2-acrylamido-2-méthylpropane sulfonique, notamment ceux se présentant sous la forme d'une émulsion E/H, tels que ceux commercialisés sous le nom de SEPIGEL 305 par la société Seppic (nom INCI : Polyacrylamide / C13-14 Isoparaffin / Laureth-7), sous le nom de SIMULGEL 600 par la société Seppic (nom INCI : Acrylamide / Sodium acryloyldimethyltaurate copolymer / Isohexadecane / Polysorbate 80),
2) les copolymères d'acide (méth)acrylique ou de (méth)acrylate et d'acide 2-acrylamido-2-méthylpropane sulfonique, notamment ceux se présentant sous la forme d'une émulsion E/H, tels que ceux commercialisés sous le nom de SIMULGEL NS par la société Seppic (copolymère d'acrylamido-2-methyl propane sulfonate de sodium / hydroxyethyl acrylate en émulsion inverse à 40% dans Polysorbate 60 et squalane) (nom INCI : hydroxyethyl acrylate/sodium acryloyldimethyltaurate copolymer / squalane / polysorbate 60) ou ceux commercialisés sous le nom de SIMULGEL EG par la société Seppic (copolymère d'acide acrylique / acrylamido-2-methylpropane-sulfonique sous forme de sel de sodium en émulsion inverse à 45% dans isohexadecane / eau) (nom INCl : Sodium Acrylate / Sodium acryloydimethyl-taurate copolymer / Isohexadecane / Polysorbate 80), ;
3) les copolymères d'acide 2-acrylamido-2-méthylpropane sulfonique et de vinylpyrrolidone ou de vinylformamide, tels que les produits commercialisés sous les dénominations ARISTOFLEX AVC par la société Clariant,
4) les copolymères d'acide 2-acrylamido-2-méthylpropane sulfonique,à motif hydrophobe, notamment les copolymères comportant un motif acide 2-acrylamido 2-méthylpropane sulfonique de formule (I) telle que définie ci-dessus, et au moins un motif hydrophobe de formule (II) 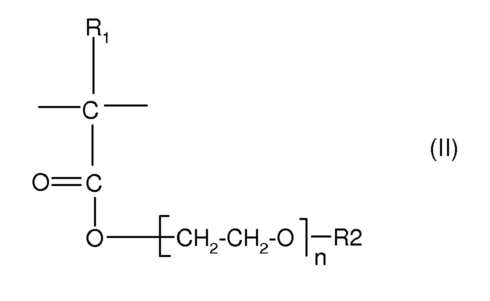
dans laquelle n désigne un nombre de moles qui est un nombre entier variant de 3 à 100, de préférence de 3 à 50 et plus préférentiellement de 7 à 25, R₁ est l'hydrogène ou un radical méthyle, et R₂ désigne un radical alkyle linéaire ou ramifié comportant de 6 à 30 atomes de carbone, de préférence de 10 à 22 atomes de carbone et mieux de 14 à 22 atomes de carbone.

Dans ces copolymères, le motif d'AMPS de formule (I) représente en général de 80 à 99 % en moles et de préférence de 85 à 99 % en moles, et le motif de formule (II) représente en général 1 à 20 % en moles et de préférence de 1 à 15 % en moles.

Comme copolymères d'AMPS à motif hydrophobe, on peut citer notamment le copolymère d'AMPS et de méthacrylate d'alcool en C₁₂-C₁₄ éthoxylé (copolymère non réticulé obtenu à partir de Genapol LA-070 et d'AMPS) (nom INCl : Ammonium Acryloyldimethyltaurate / Laureth-7 Methacrylate Copolymer) commercialisé sous la dénomination ARISTOFLEX LNC par la société Clariant, le copolymère d'AMPS et de méthacrylate de stéaryle éthoxylé (25 EO) (copolymère réticulé par le triméthylolpropane triacrylate, obtenu à partir de Genapol T-250 et d'AMPS) (nom INCl : Ammonium Acryloyldimethyltaurate / Steareth-25 Methacrylate Crosspolymer) commercialisé sous la dénomination ARISTOFLEX HMS par la société Clariant, et le copolymère d'AMPS et de méthacrylate d'alcool en C₁₆-C₁₈ éthoxylé (nom INCl : Ammonium Acryloyldimethyltaurate / Steareth-8 Methacrylate Copolymer), copolymère non réticulé obtenu à partir de Genapol T-080 et d'AMPS) commercialisé sous la dénomination ARISTOFLEX SNC par la société Clariant.

Selon un mode préféré de réalisation de l'invention, le polymère est un homopolymère d'AMPS, et notamment l'Hostacerin AMPS.

La quantité de polymère(s) d'acide 2-acrylamido 2-méthylpropane sulfonique(s) dans la composition de l'invention peut aller par exemple, en matière active, de 0,1 à 5% en poids, de préférence de 0,2 à 5 % en poids, mieux de 0,2 à 3 % en poids par rapport au poids total de la composition.

### Phase huileuse

La phase huileuse est constituée des huiles et de tous les autres corps gras et constituants lipophiles pouvant être présents dans la composition de l'invention, y compris l'élastomère de silicone et le polymère lipophile. Toutes les huiles cosmétiquement acceptables peuvent être utilisées.

On entend par "huile" un corps gras liquide à la température ambiante (25°C).

Comme huiles utilisables dans la composition de l'invention, on peut citer par exemple :
- les huiles hydrocarbonées d'origine animale (poisson), telles que le perhydrosqualène et le squalane ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stéarineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment les esters et éthers d'acides gras, comme les huiles de formules R¹COOR² et R¹OR² dans laquelle R¹ représente le reste d'un acide gras ou d'un alcool gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle, l'isononanoate d'isnonyle ; les esters hydroxylés comme l'isostéaryl lactate, l'octylhydroxystéarate, l'hydroxystéarate d'octyldodécyle, le düsostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle (Prisorine 3631) ;
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam ;
- les huiles de silicone, comme par exemple les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane et la cyclopentasiloxane ; les polydiméthylsiloxanes (ou diméthicones) comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxy-diphényl-siloxanes, les diphényl-diméthicones, les diphénylméthyldiphényl trisiloxanes, les 2-phényléthyltriméthyl-siloxysilicates, et les polyméthylphénylsiloxanes ;
- les alcools gras ayant de 8 à 26 atomes de carbone, comme l'alcool cétylique, l'alcool stéarylique et leur mélange (alcool cétylstéarylique) ;
- les huiles fluorées partiellement hydrocarbonées et/ou siliconées comme celles décrites dans le document JP-A-2-295912;
- et leurs mélanges.

On entend ci-dessus par « huile hydrocarbonée », toute huile comportant majoritairement des atomes de carbone et d'hydrogène, et éventuellement des groupements ester, éther, fluoré, acide carboxylique et/ou alcool.

Selon un mode préféré de réalisation de l'invention, la phase huileuse de la composition contient au moins 50 % en poids d'huiles non volatiles par rapport au poids total de la phase huileuse, et donc les huiles volatiles représentent au plus 50 % du poids total de la phase huileuse. Par "huile volatile", on entend une huile (ou milieu non aqueux) susceptible de s'évaporer au contact de la peau en moins d'une heure, à température ambiante et pression atmosphérique. L'huile volatile est une huile liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm de Hg), et de préférence allant de 1,3 Pa à 13 000 Pa (0,01 à 100 mm de Hg), et préférentiellement allant de 1,3 Pa à 1300 Pa (0,01 à 10 mm de Hg).

Selon un mode préféré de réalisation de l'invention, la composition contient au moins une huile choisie parmi le polyisobutène hydrogéné (nom INCI : Hydrogenated Polyisobutene ou C13-16 Isoparaffin) et les esters d'acide gras comme notamment l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle, l'isononanoate d'isnonyle, et leurs mélanges.

En plus des huiles indiquées ci-dessus, la composition de l'invention peut contenir d'autres corps gras dans la phase huileuse, tels que les acides gras comportant de 8 à 30 atomes de carbone, comme l'acide stéarique ; les résines de silicone telles que la trifluorométhyl-C1-4-alkyldimethicone et la trifluoropropyldimethicone; les gommes de silicone (dimethiconol), les élastomères de silicone non émulsionnants, comme les produits commercialisés sous les dénominations « KSG 6» ou « KSG 16» par la société Shin-Etsu, sous les dénominations «Trefil », «BY29 » ou «EPSX » par la société Dow Corning ou sous les dénominations «Gransil » par la société Grant Industries ; les cires, par exemple les cires minérales, les cires d'origine animale comme la cire d'abeille, les cires d'origine végétale, les huiles hydrogénées concrètes à 25°C, les esters gras et les glycérides concrets à 25°C, les cires synthétiques telles que la cire de polyméthylène, les cires de silicone ; et leurs mélanges. Ces différents constituants sont non volatiles et font partie de la phase huileuse.

La phase huileuse (comprenant tous les constituants lipophiles) peut être présente dans la composition selon l'invention en une quantité allant par exemple de 5 à 60 %, de préférence de 10 à 50 % en poids, mieux de 10 à 40 % en poids et mieux de 15 à 30 % en poids par rapport au poids total de la composition. Selon un mode particulièrement préféré de réalisation de l'invention, la phase huileuse représente au plus 40 % du poids total de la composition.

### Phase aqueuse

La quantité de phase aqueuse peut aller de 40 à 95 % en poids, de préférence de 50 à 90 % en poids, mieux de 60 à 90 % en poids et encore mieux de 70 à 85 % en poids par rapport au poids total de la composition. Selon un mode particulièrement préféré de réalisation de l'invention, la phase aqueuse représente au moins 60 % du poids total de la composition.

La phase aqueuse comprend de l'eau et les adjuvants (solvants, actifs et additifs) hydrophiles. L'eau représente de préférence au moins 30 % et mieux au moins 40 % du poids total de la composition, et par exemple de 30 à 95 %, de préférence de 40 à 90 % en poids et mieux de 50 à 80 % en poids par rapport au poids total de la composition.

Comme solvants hydrophiles pouvant être utilisés dans la composition de l'invention, on peut citer notamment les monoalcools comportant 2 à 8 atomes de carbone, comme l'éthanol, l'isopropanol et leurs mélanges.

### Adjuvants

De façon connue, la composition de l'invention peut contenir également des adjuvants habituels dans le domaine cosmétique, tels que les polyols, les actifs, les conservateurs, les antioxydants, les électrolytes, les agents complexants, les parfums, les charges, les bactéricides, les absorbeurs d'odeur, les matières colorantes (colorants et pigments) et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Ces adjuvants ainsi que leurs concentrations doivent être tels qu'ils ne modifient pas la propriété recherchée pour la composition de l'invention.

Comme polyols, on peut citer notamment la glycérine, les glycols comme le pentylène glycol, le propylène glycol, le butylène glycol, l'isoprène glycol et les polyéthylène glycols tels que le PEG-8 ; le sorbitol ; les sucres tels que le glucose, le fructose, le maltose, le lactose, le sucrose ; et leurs mélanges.

Comme exemples d'actifs utilisables dans la composition selon l'invention, on peut citer par exemple l'acide (N-2 hydroxyéthylpiperazine-N-2-éthane) sulfonique (HEPES) ; l'acide hyaluronique ; la lanoline ; l'urée, les mélanges contenant de l'urée, tels que le NMF (« Natural Moisturising Factor»), et les dérivés d'urée tels que le N-(2-hydroxyéthyl)-urée (Hydrovance de la société National Starch) ; l'acide 2-oxothiazolidine-4-carboxylique (procystéine) ; les α-hydroxy-acides notamment les acides dérivés de fruits, comme les acides glycolique, lactique, malique, citrique, tartrique, mandélique, leurs dérivés et leurs mélanges ; les β-hydroxy-acides comme l'acide salicylique et ses dérivés tels que l'acide n-octanoyl-5-salicylique ou l'acide n-dodécanoyl-5 salicylique ; les α-céto-acides comme l'acide ascorbique ou vitamine C et ses dérivés tels que ses sels comme l'ascorbate de sodium, l'ascorbylphosphate de magnésium ou de sodium ; ses esters comme l'acétate d'ascorbyle, le palmitate d'ascorbyle et le propionate d'ascorbyle, ou ses sucres comme l'acide ascorbique glycosylé, et leurs mélanges ; les β-céto-acides ; les rétinoïdes comme le rétinol (vitamine A) et ses esters, le rétinal, l'acide rétinoïque et ses dérivés, ainsi que les rétinoïdes décrits dans les documents FR-A-2,570,377, EP-A-199 636, EP-A-325 540, EP-A-402 072,EP-A-325 540, EP-A-402 072 ; les caroténoïdes tels que le lycopène ; les céramides ; les sapogénines et les extraits végétaux en contenant, en particulier les extraits de Wild Yam ; le resvératrol ; les pseudodipeptides tels que l'acide {2-[acétyl-(3-trifluorométhyl-phényl)-amino]-3-méthyl-butyrylamino} acétique ; les vitamines comme par exemple, outre les vitamines A et C indiquées ci-dessus, la vitamine E (tocophérol), la vitamine B3 (ou vitamine PP ou niacinamide), la vitamine B5 (panthénol sous ses différentes formes : D-panthénol, DL-panthénol), la vitamine D, la vitamine F (mélange d'acides gras essentiels), les dérivés, précurseurs et analogues de ces vitamines ; les extraits de soja, en particulier les hydrolysats de protéine de soja ou les extraits de soja riches en isoflavones ; les oligo-éléments comme le cuivre, le zinc, le sélénium, le fer, le magnésium, le manganèse ; les extraits d'algues comme le produit commercialisé sous la dénomination Stimoderm par la société CLR ; les extraits de plancton tels que le plancton en dispersion aqueuse (nom lNCl : Vitreoscilla Ferment) commercialisé sous la dénomination Mexoryl SAH par la société Chimex ; les enzymes ; les coenzymes tels que l'ubiquinone ou coenzyme Q10 qui appartient à la famille des benzoquinones à chaîne alkylénée, le coenzyme R qui est la biotine (ou vitamine H) ; les extraits de levure comme l'extrait de s.cerevisiae commercialisé sous la dénomination Cytovitin LS 9388 par les laboratoires Sériobiologiques; l'adénosine ; les extraits végétaux comme l'extrait de réglisse ; les agents apaisants comme le bisabolol et les extraits végétaux apaisants comme les extraits de rose (rosa gallica) et les extraits de menthe (mentha piperita) ; les dérivés C-glycosides tels que C-β-D-xylopyranoside-n-propane-2-one, par exemple sous forme de solution à 30 % en matière active dans un mélange eau/propylèneglycol (60/40 % en poids) tel que le produit fabriqué par la société Chimex sous la dénomination commerciale Mexoryl SBB^{®} ; et tout actif approprié pour le but final de la composition, et leurs mélanges.

La composition peut contenir une ou plusieurs charges. Comme charges, la composition de l'invention peut contenir par exemple des particules minérales telles que les argiles, les silices, les oxydes métalliques tels que le dioxyde de titane ou l'oxyde de zinc, le mica, et/ou des charges organiques telles que les particules de polyamide (Nylon) et notamment celles vendues sous les dénominations ORGASOL par la société Atochem ; les poudres de polyéthylène ; les microsphères à base de copolymères acryliques, telles que celles en copolymère diméthacrylate d'éthylène glycol/ méthacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; et leurs mélanges. La quantité de charge(s) peut aller par exemple de 0,05 à 10 % en poids et mieux 0,1 à 5 % en poids par rapport au poids total de la composition.

La composition selon l'invention peut se présenter avantageusement sous forme de crème plus ou moins fluide, c'est-à-dire un produit ayant une certaine consistance mais souple par opposition à un produit solide tel qu'un stick. Ainsi, cette composition peut avoir une viscosité à 25°C allant d'environ 10 à 200 poises (1 à 20 Pa.s), de préférence d'environ 15 à 150 poises (1,5 à 15 Pa.s) et mieux de 20 à 100 poises (2 à 10 Pa.s), cette viscosité étant mesurée avec un appareil Rhéomat 180 à 25°C avec les mobiles appropriés.

La composition cosmétique selon l'invention trouve son application dans un grand nombre de traitements cosmétiques des matières kératiniques, et plus spécialement de la peau, en particulier dans le soin de la peau, pour nourrir la peau, prévenir la perte d'eau transépidermique et protéger la peau.

Aussi, la présente invention a pour objet l'utilisation cosmétique de la composition telle que définie ci-dessus, pour le soin de la peau, en particulier pour nourrir la peau, prévenir la perte d'eau transépidermique et/ou protéger la peau.

La composition est particulièrement appropriée pour le traitement de la peau sèche.

La présente invention a aussi pour objet un procédé cosmétique de traitement de la peau sèche, comprenant l'application sur la peau, de la composition telle que définie ci-dessus.

Les exemples ci-après de compositions selon l'invention sont donnés à titre d'illustration et sans caractère limitatif. Les composés sont indiqués en nom chimique ou en nom INCI. Les quantités y sont données en % en poids de matière première, sauf mention contraire.

### Exemples 1 et 2 selon l'invention et exemple comparatif 1

| Composition | Exemple 1 selon l'invention | Exemple 2 selon l'invention | Exemple comparatif 1 |
|---|---|---|---|
| Phase aqueuse | | | |
| Glycérine | 7 | 7 | 7 |
| Hostacerin AMPS | 1 | 1 | 1 |
| Conservateur | 0,4 | 0,4 | 0,4 |
| Eau | qsp 100 % | qsp 100 % | qsp 100 % |
| Phase huileuse | | | |
| KSG210 à 25% en matière active (soit 1,65 % de M.A.) | 6,6 | 6,6 | 6,6 |
| Homopolymère d'acrylate de béhényle (Intelimer IPA-13.6) | 2 | 2 | 0 |
| Cyclohexamethicone | 5 | 3 | 7 |
| Polyisobutène hydrogéné (Huile de Parleam) | 5 | - | 5 |
| Isohexadécane | 5 | 5 | 5 |
| Résultat en stabilité (2 mois à 45°C) | Stable | Stable | Instable |

Mode opératoire : chaque phase a été homogénéisée et chauffée à 65°C. Puis on a versé lentement la phase aqueuse dans la phase huileuse.

Les compositions de l'invention se présente sous forme de crème et sont stables, contrairement à la composition de l'exemple comparatif ne contenant pas de polymère semi-cristallin.

### Exemple 3 selon l'invention

| *Phase aqueuse* | |
|---|---|
| Conservateur | 0,4 % |
| Polymère d'AMPS (Hostacerin AMPS) | 1 % |
| Eau | Qsp 100 % |
| | |
| *Phase huileuse* | |
| KSG 210 (à 25 % en matière active) | 3 % |
| (soit 0,75 % de matière active) | |
| Homopolymère d'acrylate de stéaryle (Intelimer IPA-13.1) | 2 % |
| Polyisobutène hydrogéné (Huile de Parleam) | 10 % |
| Isohexadécane | 5 % |

Le mode opératoire est identique à celui indiqué pour les exemples précédents.
On a obtenu une crème épaisse, homogène et lisse, dont la viscosité était de 80 poises (soit 8 Pa.s) (mesure faite à 25°C, 10 minutes après préparation, au Rhéomat 180, mobile 4). Cette crème était fraîche à l'application tout en étant nutritive. Au microscope, l'émulsion était régulière avec des bords nets. La composition est restée stable après le test de centrifugation.

### Exemple comparatif 2 :

| *Phase aqueuse* | |
|---|---|
| Conservateur | 0,4 % |
| Polymère d'AMPS (Hostacerin AMPS) | 1 % |
| Eau | Qsp 100 % |
| | |

| *Phase huileuse* | |
|---|---|
| KSG 210 (à 25 % en matière active) | 3 % |
| (soit 0,75 % de matière active) | |
| Polyisobutène hydrogéné (Huile de Parleam) | 10 % |
| Isohexadécane | 5 % |

Le mode opératoire est identique à celui indiqué pour les exemples précédents.
On a obtenu un produit plus fluide que celui de l'exemple 3 selon l'invention, sa viscosité étant de 14 poises (soit 1,4 Pa.s) (mesure faite à 25°C, 10 minutes après préparation, au Rhéomat 180, mobile 3). En outre, le produit était moins lisse et d'aspect plus hétérogène que celle de l'exemple 3 selon l'invention. De plus, au microscope, l'émulsion était moins régulière avec des bords lâches et des plages de relargage. En outre, cette émulsion s'est révélée instable au test de centrifugation car elle s'est déphasée. Cet exemple comparatif montre qu'en absence de polymère semi-cristallin, la composition n'est plus une crème et qu"elle est moins stable.

### Exemple 4 selon l'invention : Crème hydratante de jour

| *Phase aqueuse* | |
|---|---|
| Conservateur | 0,4 % |
| Glycérine | 5 % |
| Polymère d'AMPS (Hostacerin AMPS) | 1 % |
| Eau | Qsp 100 % |
| | |
| *Phase huileuse* | |
| KSG 210 (à 25 % en matière active) | 4 % |
| (soit 1 % de matière active) | |
| Homopolymère d'acrylate de stéaryle (Intelimer IPA-13.1) | 1 % |
| Isohexadécane | 5 % |
| Cyclohexaméthicone | 3 |
| Polyisobutène hydrogéné (Huile de Parleam) | 7 % |

Le mode opératoire est identique à celui indiqué pour les exemples précédents.
On a obtenu une crème assez épaisse, fondante sur la peau ; elle était fraîche et très agréable à utiliser. Après application de cette crème, la peau était douce, fraîche et bien hydratée.

### Exemple 5 selon l'invention : Crème pour la nuit

| *Phase aqueuse* | |
|---|---|
| Conservateur | 0,4 % |
| Glycérine | 5 % |
| Polymère d'AMPS (Hostacerin AMPS) | 1 % |
| Eau | Qsp 100 % |
| | |

| *Phase huileuse* | |
|---|---|
| KSG 710 (à 25 % en matière active) | 8 % |

| (soit 2 % de matière active) | |
|---|---|
| Homopolymère d'acrylate de stéaryle (Intelimer IPA-13.1) | 2 % |
| Isohexadécane | 5 % |
| Polyisobutène hydrogéné (Huile de Parleam) | 10 % |

Le mode opératoire est identique à celui indiqué pour les exemples précédents.

On a obtenu une émulsion fine, fraîche et pourtant nutritive à l'application, qui s'étalait facilement et a été rapidement absorbée dans la peau qu'elle a laissée, apaisée, lissée et non grasse.

### Exemple 6 selon l'invention et exemple comparatif 3

| Composition | Exemple 6 selon l'invention | Exemple comparatif 3 |
|---|---|---|
| Phase aqueuse | | |
| Glycérine | 5 | 5 |
| Sulfate de magnésium | 0,7 | 0,7 |
| Conservateur | 0,4 | 0,4 |
| Eau | qsp 100 % | qsp 100 % |

| Phase huileuse | | |
|---|---|---|
| KSG210 à 25% en matière active (soit 4 % de M.A.) | 16 | 16 |
| Homopolymère d'acrylate de béhényle (Intelimer IPA-13.6) | 1 | 0 |
| Huile de jojoba | 5 | 5 |
| Cire de polymèthylène | 2 | 2 |
| Cyclohexamethicone | 5 | 5 |
| Polyisobutène hydrogéné (Huile de Parleam) | 10 | 10 |
| Aspect au microscope après 5 jours | Stable | Plages de relargage d'huiles |

Le procédé de préparation a été le même que pour les autres exemples. L'exemple 6 constitue une crème qui est adaptée au soin des peaux très sèches. L'exemple comparatif 3 est plus fluide et est moins stable, malgré la présence d'épaississant de phase huileuse (cire) et la présence de sel dans la phase aqueuse (sulfate de magnésium).

### Exemple 7 selon l'invention : Crème hydratante

| *Phase aqueuse* | |
|---|---|
| Conservateur | 0,4 % |
| Glycérine | 7 % |
| Sulfate de magnésium | 0,7 % |
| Eau | Qsp 100 % |

| *Phase huileuse* | |
|---|---|
| KSG 710 (à 25 % en matière active) | 16 % |
| (soit 4 % de matière active) | |
| Homopolymère d'acrylate de stéaryle (Intelimer IPA-13.1) | 1 % |
| Polyisobutène hydrogéné (Huile de Parleam) | 15 % |
| Cire de polymèthylène | 1 % |
| Cyclohexasiloxane | 5 % |
| | |
| Charges | |
| Amidon de mais estérifié par anhydride octenyl succinique | |
| (Dry Flo) | 3 % |

On obtient une crème onctueuse, riche, confortable, qui laisse la peau mate, non grasse, hydratée.

## Revendications

1. Composition pour application topique sous forme d'émulsion eau-dans-huile comprenant une phase aqueuse dispersée dans une phase huileuse, et contenant au moins un élastomère de silicone émulsionnant et au moins un polymère semi-cristallin.

2. Composition selon la revendication 1, **caractérisée en ce que** l'élastomère de silicone émulsionnant est choisi parmi les élastomères de silicone comportant au moins une chaîne oxyalkylénée et/ou une chaîne glycérolée.

3. Composition selon la revendication 2, **caractérisée en ce que** l'élastomère de silicone comportant au moins une chaîne oxyalkylénée est obtenu par réaction d'addition et réticulation de diorganosiloxane contenant au moins un hydrogène lié au silicium et un polyoxyalkylène ayant au moins deux groupements à insaturation éthylénique, en présence de catalyseur.

4. Composition selon l'une quelconque des revendications précédentes, **caractérisée en ce que** l'élastomère de silicone émulsionnant est un élastomère de silicone comportant au moins une chaîne oxyéthylénée.

5. Composition selon la revendication 2, **caractérisée en ce que** l'élastomère de silicone comportant au moins une chaîne glycérolée est obtenu par réaction d'addition et réticulation de diorganosiloxane contenant au moins un hydrogène lié au silicium et de composés polyglycérolés ayant des groupements à insaturation éthylénique, en présence de catalyseur.

6. Composition selon l'une quelconque des revendications précédentes, **caractérisée en ce que** la quantité d'élastomère(s) de silicone émulsionnant(s) va de 0,1 à 20 % en poids par rapport au poids total de la composition.

7. Composition selon l'une quelconque des revendications précédentes, **caractérisée en ce que** le polymère semi-cristallin est choisi parmi les homopolymères et copolymères résultant de la polymérisation d'au moins un monomère à chaîne(s) cristallisable(s), celle-ci étant choisie parmi les chaînes alkyles comportant au moins 11 atomes de carbone et au plus 40 atomes de carbone.

8. Composition selon l'une quelconque des revendications précédentes, **caractérisée en ce que** le polymère semi-cristallin est choisi parmi les homopolymère obtenus par polymérisation d'un monomère choisi parmi les acrylates d'alkyle en C₁₄-C₂₄ et les méthacrylates d'alkyle en C₁₄-C₂₄ et parmi les copolymères obtenus par copolymérisation d'un monomère choisi parmi les acrylates d'alkyle en C₁₄-C₂₄ et les méthacrylates d'alkyle en C₁₄-C₂₄, avec un monomère hydrophile.

9. Composition selon l'une quelconque des revendications précédentes, **caractérisée en ce que** le polymère semi-cristallin est choisi parmi l'homopolymère d'acrylate de stéaryle, l'homopolymère d'acrylate de béhényle, et leurs mélanges.

10. Composition selon l'une quelconque des revendications précédentes, **caractérisée en ce que** la quantité de polymère(s) semi-cristallin(s) va de 0,1 à 5 % en poids par rapport au poids total de la composition.

11. Composition selon l'une quelconque des revendications précédentes, **caractérisée en ce qu'**elle contient en outre un ou plusieurs polymères d'acide 2-acrylamido 2-méthylpropane sulfonique.

12. Composition selon la revendication précédente, **caractérisée en ce que** le polymère d'acide 2-acrylamido 2-méthylpropane sulfonique est un homopolymère comprenant, distribués de façon aléatoire, des motifs de formule générale (I) suivante : 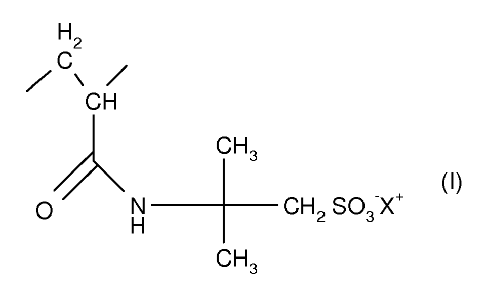 dans laquelle X⁺ désigne un proton, un cation de métal alcalin, un cation alcalino-terreux ou l'ion ammonium, au plus 10% mol des cations X⁺ pouvant être des protons H⁺ ;
et des motifs réticulants provenant d'au moins un monomère ayant au moins deux double-liaisons oléfiniques.

13. Composition selon la revendication 11, **caractérisée en ce que** le polymère d'acide 2-acrylamido 2-méthylpropane sulfonique est un copolymère choisi parmi :
- les copolymères anioniques réticulés d'acrylamide ou méthylacrylamide et d'acide 2-acrylamido-2-méthylpropane sulfonique,
- les copolymères d'acide(méth)acrylique ou de (méth)acrylate et d'acide 2-acrylamido-2-méthylpropane sulfonique,
- les copolymères d'acide 2-acrylamido-2-méthylpropane sulfonique et de vinylpyrrolidone ou de vinylformamide,
- les copolymères comportant un motif acide 2-acrylamido 2-méthylpropane sulfonique de formule (I), et au moins un motif hydrophobe de formule (II) 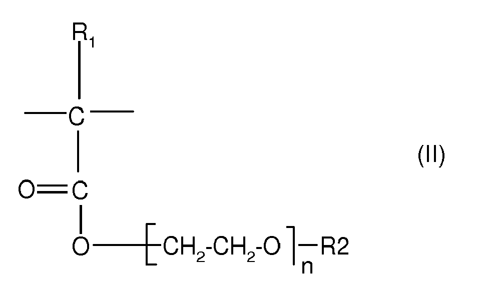
dans laquelle n désigne un nombre entier variant de 3 à 100, ; R₁ est l'hydrogène ou un radical méthyle, et R₂ désigne un radical alkyle linéaire ou ramifié comportant de 6 à 30 atomes de carbone.

14. Composition selon l'une quelconque des revendications 11 à 13, **caractérisée en ce que** la quantité de polymère(s) d'acide 2-acrylamido 2-méthylpropane sulfonique(s) va de 0,1 à 5 % en poids par rapport au poids total de la composition.

15. Composition selon l'une quelconque des revendications précédentes, **caractérisée en ce que** la phase huileuse comprend au plus 50 % d'huiles volatiles par rapport au poids total de la phase huileuse.

16. Composition selon l'une quelconque des revendications précédentes, **caractérisée en ce qu'**elle contient au moins une huile choisie parmi le polyisobutène hydrogéné, les esters d'acide gras, et leurs mélanges.

17. Composition selon l'une quelconque des revendications précédentes, **caractérisée en ce que** la phase aqueuse est présente en une quantité allant de 40 à 95 % par rapport au poids total de la composition.

18. Composition selon l'une quelconque des revendications précédentes, **caractérisée en ce qu'**elle contient au moins 30 % d'eau en poids par rapport au poids total de la composition.

19. Utilisation cosmétique d'une composition selon l'une quelconque des revendications 1 à 18, pour le soin de la peau.

20. Utilisation cosmétique d'une composition selon l'une quelconque des revendications 1 à 18, pour nourrir la peau, prévenir la perte d'eau transépidermique et/ou protéger la peau.

21. Procédé cosmétique de traitement de la peau sèche, comprenant l'application sur la peau, d'une composition selon l'une quelconque des revendications 1 à 18.
